(19) Europäisches Patentamt European Patent Office Office européen des brevets

(11) **EP 3 273 428 B1**

(12) **EUROPEAN PATENT SPECIFICATION**

(45) Date of publication and mention of the grant of the patent:
**28.08.2019   Bulletin 2019/35**

(51) Int Cl.:
***G09B 19/00*** *(2006.01)*

(21) Application number: **16180006.5**

(22) Date of filing: **18.07.2016**

(54) **METHOD AND APPARATUS FOR EVALUATING THE QUALITY OF CHARACTERISTICS OF MOTION OF AN ANIMAL-HUMAN-DYAD**

VERFAHREN UND VORRICHTUNG ZUR QUALITÄTSBEURTEILUNG VON EIGENSCHAFTEN EINER TIER-MENSCH-DYADE

PROCÉDÉ ET APPAREIL POUR ÉVALUER LA QUALITÉ DES CARACTÉRISTIQUES DU MOUVEMENT D'UN DUO ANIMAL-HUMAIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**24.01.2018   Bulletin 2018/04**

(73) Proprietor: **Avansce Ltd.**
**Bishop Auckland**
**Durham DL14 7JH (GB)**

(72) Inventor: **Cross, Graham Hugh**
**Stockton-on-Tees,**
**North Yorkshire TS21 1HN (GB)**

(74) Representative: **Flügel Preissner Schober Seidel**
**Patentanwälte PartG mbB**
**Nymphenburger Strasse 20a**
**80335 München (DE)**

(56) References cited:
**NZ-A- 540 119          US-A- 5 369 601**
**US-A1- 2015 106 052**

**Description**

The invention relates to a method and an apparatus for evaluating the quality of characteristics of motion of an animal-human-dyad comprising an animal and a human and using at least one rein for riding the animal. In particular, the invention relates to a horse-rider-dyad that means the interaction between a horse and a rider positioned on the horse. Moreover, the present invention relates to a rein tension measuring device comprising a rein tension sensor unit and at least one attachment unit for fastening the rein tension sensor unit to the rein.

Unlike any other sporting activity the riding by humans of other animals is unique in that success relies upon the mutual acceptance of a continuous physical connection between those two otherwise independent animals each having its own free will.

The human and the animal having a continuous physical connection between them is together called the animal-human dyad.

Riding of animals by humans requires a physical connection to the animal through the seat of the human and through the lower legs of the human in most cases also.

Riding of some animals by humans requires a physical connection from the human's hands to the animal through a mechanical system of restraint such as reins and bridlework attached to the animal's head with sometimes a mechanical device supported by the reins and bridlework in the animal's mouth.

The mass of the animal being ridden is invariably much larger than that of the human and thus its contribution to the total momentum of the animal/human dyad is dominant.

Without some method of physical restraint or means of communication from the human to the animal the animal-human-dyad would follow the paths of motion and speed of motion determined by the animal's own free will.

One example of an animal/human dyad is the horse-rider-dyad (HRD).

In the horse-rider-dyad methods of physical restraint usually include reins attached to bridlework on the horse's head and also more usually a mechanical device known as a bit in the horse's mouth.

Commonly accepted preferred methods of riding of horses by riders require humane approaches to the physical restraint that do not cause harm to the horse.

Even more preferred methods require an understanding by the rider of the capabilities and limitations of the horse and in consequence the application of the minimum physical restraint necessary to direct the paths of motion and speeds of motion of the dyad as determined by the rider's own free will.

The riding of horses by humans in a competitive context is the equestrian sports.

Preferred in some equestrian sports is that the horse and rider appear to exhibit desirable visual characteristics as defined and scored by judges of the governing bodies that define and organise the equestrian sports.

Experienced riders on horses that are fully fit mentally and physically are able to succeed in such competitions using means of communication and physical restraint that are appropriate as evidenced by the visual characteristics of motion of the horse-rider-dyad observed by judges of the governing bodies.

However it is also possible that the desirable visual characteristics exhibited by the horse-rider-dyad are produced by inhumane approaches to the physical restraint and poor means of communication from the human to the horse.

It is also possible that amongst those judges of the governing bodies there might be subjective differences between them in scoring the quality of the characteristics of motion of the horse-rider-dyad and it is therefore usual to pool the scores of a plurality of those judges to obtain the average score.

In some competitions there is only one judge in attendance and no averaging is possible.

The consequence of making judgements of the quality of the characteristics of motion of the horse-rider-dyad from visual observations is that inhumane approaches to the physical restraint and poor means of communication from the human to the horse could remain undiscovered and further that where averaging of the scores from a plurality of judges is not possible those judgements cannot be made objective in addition to the possibility that the inhumane approaches to the physical restraint and poor means of communication from the human to the horse could remain undiscovered.

In most other sports even including some equestrian sports there is an objective judgement of quality of performance such as a measure of distance or time and instruments have been developed to report these quantities so that subjective judgement is not required.

Particularly in the sports involving the riding of animals there is a desire to determine the level of fitness and physical condition of the animal during competitive activity. With those four legged animals that are ridden the presence of lameness in one or more limbs presents as an unevenness and asymmetry in the right and left phases of each single full stride a condition known as unlevelness. It is often difficult to detect such physical unevenness yet it is desirable to do so from the viewpoint of animal welfare. The general definition of this is laterality.

US 5,369,601 A and NZ 540,119 A each refer to an apparatus for equestrian monitoring for instruction and training. To this end, the force applied by the rider to the horse is measured. A strain gauge is located in the reins in order to measure the tension in the reins applied by the rider. The measured force is displayed to an observer. US 2015/0106052 A1 refers to a step counter which has an accelerometer for detecting a step or other movements by a person. For analyzing the data generated by the accelerometer, a Fourier analysis is applied.

It is an object of the present invention to provide a method and an apparatus for obtaining an objective score of the quality of the characteristics of motion of the dyad.

## Summary of the invention

The above object is achieved by a method according to claim 1.

According to the present invention, a method for evaluating the quality of characteristics of motion of an animal-human-dyad comprising an animal and a human and using at least one rein for riding the animal comprises the following step, wherein the quality of characteristics of motion is the loss of regularity of rhythm in the motion of the dyad: obtaining rein tension data indicating the tension in the rein by means of a rein tension measuring device. The following steps are executed by a processing unit: processing the rein tension data into a frequency spectrum; identifying a prominent frequency component in the frequency spectrum; analysing the frequency spectrum by determining a power spectral density of the frequency spectrum over a frequency range which contains the prominent frequency component; and evaluating the loss of regularity in the motion by comparing the determined power spectral density with a reference value.

As to the understanding of the invention, the method can preferably be understood as a method or system for identifying the degree of acceptance by a horse of commands given to it by a rider via the reins and acceptance by the rider of the forces in response from the horse. The method includes measuring the dynamic characteristics of those pairwise forces and to quantify the degree of mutual coherence and mutual acceptance between the reactions of the horse and the actions of the rider.

When comparing the determined power spectral density with a reference preferably a ratio thereof is determined and reported as a figure of merit of the quality of the characteristics of the motion.

Furthermore, as to the terms used in the present invention, the prominent frequency component can also be designated as main frequency component or peak frequency. The frequency range of the analysed frequency spectrum which contains the prominent frequency component can be defined by a lower frequency limit and an upper frequency limit with the frequency range between. The reference value preferably is a predetermined value or range of reference values or map of reference values.

Preferred embodiments of the method of the invention are defined in claims 2 to 9 and will be further explained below.

In a preferred embodiment a signal-to-noise ratio (s/n) is determined by analysing the frequency spectrum.

According to a preferred embodiment the frequency spectrum is analysed within a frequency sub-range spanning the prominent frequency component. In other words, preferably, the frequency data within the frequency range and the frequency data within the frequency sub-range are analysed.

In a preferred embodiment the frequency sub-range is centered on the prominent frequency component.

In a further preferred embodiment, the proportion of the power spectral density is determined over the frequency sub-range, which in particular can be centred on the prominent frequency component, and is compared to that calculated over the frequency range to either side of that prominent frequency component. The proportion of the power spectral density calculated over the frequency sub-range compared to that calculated over the frequency range to either side of the prominent frequency component is reported to the rider as a measure of both their riding skill and the level of fitness and ability of their horse. Rider and horse can then follow a programme of training that maximises that proportion.

In a preferred embodiment the frequency sub-range spans the prominent frequency component such that the frequency sub-range is centered on the prominent frequency component. Preferably the frequency sub-range lies within the frequency range and in within the overall frequency range. Preferably, the frequency sub-range is limited by a first sub-range threshold and a second sub-range threshold.

As to the terms used above regarding the different ranges of frequencies, the overall frequency range can be designated as first frequency range, the frequency range can be designated as second frequency range and the frequency sub-range can be designated as third frequency range.

The values for the lower and upper limits of the frequency range and the frequency sub-range are chosen according to the mode of motion of the animal. Preferably, amongst the various modes of motion of 4 legged animals, in particular horses, are the modes walk, trot and canter.

Preferably, for walk the lower and upper limits of the frequency range are around 2.0 Hz and 5.0 Hz, preferably around 2.6 Hz and 4.6 Hz, respectively. Preferably, for trot the lower and upper limits of the frequency range are around 2.0 Hz and 5.0 Hz, preferably around 2.4 Hz and 4.4 Hz, respectively. Preferably, for canter the lower and upper limits of the frequency range are around 1.0 Hz and 2.5 Hz, preferably around 1.2 Hz and 2.2 Hz, respectively.

Preferably, the frequency range is between about 1.0 Hz to about 2.0 Hz, in particular around 1.4 Hz. Preferably, the frequency sub-range is between about 0.4 Hz to about 0.8 Hz, in particular around 0.6 Hz. However, other values are possible.

In a preferred embodiment the frequency spectrum is analysed only within an overall frequency range limited by a first frequency threshold and a second frequency threshold. Preferably, the first frequency threshold amounts to about 0.5 Hz and the second frequency threshold amounts to about 5 Hz. In other words, the overall frequency spectrum is cut off outside the overall frequency range.

In a preferred embodiment a signal-to-noise ratio (s/n) is determined for analysing the frequency spectrum, the frequency range and/or the frequency sub- range. Preferably, the normalised signal-to-noise ratio is determined.

In a preferred embodiment the signal-to-noise ratio of at least the prominent frequency component is determined.

In a preferred embodiment the signal-to-noise ratio of two or more prominent frequency components are determined and a ratio of their values is determined.

According to a preferred embodiment the reference value is the prominent frequency component.

In a preferred embodiment the reference value is a value that correlates to the stride frequency or gait frequency of the animal.

In a preferred embodiment the reference value is a value that correlates to the second harmonic of the stride frequency or gait frequency of the animal.

According to a preferred embodiment obtaining rein tension data indicating the tension in the rein comprises measuring the rein tension by at least one strain gauge inserted into the at least one rein.

According to a preferred embodiment an average of the rein tension is determined using the rein tension data and comparing with a reference value or reference table of rein tension values.

In a preferred embodiment, the step of comparing includes determining a ratio of the power spectral density and the reference value.

Preferably, the data is analysed by using Fourier Transform.

According to a further preferred embodiment of the method, the system comprises strain gauges inserted in the lines of the reins on both sides, collection of dynamic data sent by wireless transmission to a receiving device at which the dynamic data is analysed to find a dominant contribution to the frequency spectrum, e.g. in the sub-5Hz frequency region, and the proportion of power spectral density calculated over a small range of frequencies centred on the prominent frequency component is compared to that calculated over a wider range to either side of that prominent frequency component. The proportion of the power spectral density calculated over the smaller range of frequencies compared to that calculated over the frequency range to either side of the prominent frequency component is reported to the rider as a measure of both their riding skill and the level of fitness and ability of their horse. Rider and horse can then follow a programme of training that maximises that proportion.

In a preferred embodiment the method comprises the calculation of the power spectral density of the time-series rein tension data and the signal-to-noise ratio of a suitable prominent frequency in the spectrum and the reporting of that as a measure of the quality of the characteristics of motion of the animal/rider dyad.

Preferably, the method comprises the calculation of the signal-to-noise ratios of two suitable prominent frequencies in a single spectrum and the calculation of their ratio reported as a measure of laterality in the characteristics of motion of the animal/rider dyad. Preferably, the normalised signal-to-noise ratio is calculated.

A further preferred embodiment of the method is the calculation of the average of the time series rein tension and comparison against a look-up table providing a figure of merit that is a maximum for a range of average rein tensions centred on an optimum value and then being reduced to zero when the comparison is made at points further distant higher and lower to either side of this optimum value.

As a further method the ratio of two signal-to-noise ratios of two prominent peaks in the power spectral density spectrum of a single time-series data may be reported as a measure of laterality in the characteristics of motion of the animal/human dyad.

In further preferred methods the signal-to-noise figure of merit may be combined with other figures of merit that are obtainable from further computation of the time-series data available from obtaining the rein tension data.

One such method is based on a figure of merit that compares the average tension in the reins as computed from the time-series data with values in a look-up table or similar that defines an optimum value of average rein tension. A further method is based on the computation of a figure of merit that is the ratio of the average rein tension between the left and right sides of the animal where the optimum value of the figure of merit is achieved when the two values are equal.

A preferred embodiment of the method includes the following steps: rein tension data is taken in real-time, e.g. at 10 ms intervals, from both reins. The data is converted into a frequency spectrum using Fourier Transform. The main frequency component that correlates to the stride frequency or second harmonic of the stride frequency of the horse appears in this spectrum as a prominent feature below a reference value, e.g. 5 Hz, is identified. If the rider and horse have good coherence between them in their connection through the reins the size and resolution of this prominent feature is large and vice versa.

In this way, it is possible to measure the prominence of this feature through calculation of the signal to noise ratio (s/n). When the signal to noise ratio is high the rider is skilled and the horse is fit. When the signal to noise ratio is low either the rider is unskilled and the horse is fit or the horse is unfit and the rider is skilled, or both the rider is unskilled and the horse is unfit.

According to a further aspect of the present invention, the above object is achieved by an apparatus according to claim 10.

According to the present invention, an apparatus for evaluating the quality of characteristics of motion of an animal-human-dyad comprising an animal an a human and using at least one rein for riding the animal, wherein the quality of characteristics of motion is the loss of regularity of rhythm in the motion of the dyad, comprises: at least one rein tension measuring device configured to obtain rein tension data; a transmitter unit configured to transmit the rein tension data; a receiving unit configured to receive the data; a processing unit configured to process the rein tension data into a frequency spectrum, to identify a prominent frequency component in the frequency spectrum, to analyse the frequency spectrum by determining the power spectral density over a frequency range which contains the prominent frequency, and to evaluate the loss of regularity of rhythm in the motion by comparing the determined power spectral density with a reference value, and a notification unit for notifying about the loss of regularity of rhythm in the motion based on the processed data.

According to a preferred embodiment the processing unit is configured to process the data according to any of the claims 1 to 9.

In a preferred embodiment the apparatus comprises two rein tension measuring device, e.g. force transducers or strain gauges, one of them disposed on a first (left) rein and the other one disposed on a second (right) rein. In this way, rein tension data of the left-side and the right-side rein of a horse can b obtained. This enables to compare the left side forces and the right side forces.

In a preferred embodiment the at least one rein tension measuring device is embedded in the rein. Thus, the above preferred methods may be applied to either of the left or right side time-series data of the force transducers embedded in the left-side rein and right-side rein. This has the advantage that it can be determined whether there is a laterality is on the left or the right side of the horse.

In a preferred embodiment an electric cable for transmitting signals from the rein tension measuring device to the transmitting unit is embedded in the rein.

Preferably, the receiving unit, the processing unit and the notification unit are formed by one electronic device separated and remote from the rein tension measuring device. Thus, the electronic device can be used by the rider, a trainer or a judge. Further, the transmitting unit can be separated and remote from the rein tension measuring device, e.g. positioned at the animal or at the rider. Alternatively, the transmitting unit can be included into the electronic device.

As to the understanding of the apparatus of the invention, the apparatus can comprise a rein tension measuring device like a force transducer in the reins of an animal ridden by a human which sends time-series signals to a wireless transceiver and then signals are processed by the above method for viewing by the user.

Preferably, the force transducers are embedded in the material of the reins to become unobtrusive and substantially hidden and the signals therefrom are processed by a method that calculates the signal-to-noise (s/n) ratio of a prominent peak in the power spectral density spectrum of time-series data.

The signal-to-noise ratio is reported as a figure of merit of the quality of the characteristics of motion of the animal/human dyad.

Preferably, the notification unit is a display unit. This display unit can indicate the figure of merit and/or other features for showing the quality of the characteristics of motion of the animal/human dyad.

According to a further aspect, a rein tension measuring device comprises a rein tension sensor unit and at least one attachment unit for fastening the rein tension sensor unit to the rein, wherein the sensor unit is embedded in the material of the rein.

According to a preferred embodiment the attachment unit is embedded in the material of the rein. The rein tension sensor unit can be placed between the attachment units.

Preferably, the attachment units can be provided with holes for fastening to the reins by a suitable fastener. Preferably, each end of the rein tension sensor unit is provided with suitable mechanical attachment units with holes so that it can be attached to the construction material of the reins using a suitable fastener. A preferred fastener is a rivet fastener but alternatives are possible.

In a preferred embodiment an electric cable for transmitting signals is embedded in the material of the rein.

Preferably, the electric cable is provided for transmitting signals from the rein tension sensor unit to a transmitter unit. Also preferred the electrical cable is hidden from view by being fully encased in the construction materials of the reins.

In a preferred embodiment the rein tension sensor unit is a force transducer or strain gauge.

Preferably, the rein tension measuring device is inserted into each of two reins one on each side of the animal so that a time-series signal can be measured from each which is proportional to the time-series rein tension on each side of the animal. Preferably, the rein tension sensor units are hidden from view by being fully encased in the construction material of the reins 10. Preferably, also the attachment units are embedded and encased in the material of the reins.

It is known that force transducers can be mounted to the reins but with the disadvantage of visual discontinuity.

The transducers of the prior art attach to the reins or bridlework using clips or hooks and are designed for temporary use. These items make regular use of the transducers difficult and further distract from the desirable visual continuity of traditional reins and bridlework. A further disadvantage is that the electrical cables which are necessary to carry signals from the force transducer to the transmitter are also visible and obtrusive and may become trapped in the bridlework or by the rider's hands during riding.

Preferably, the rein tension sensor unit and electric cable is of a small form factor and consequently of light weight so that it can be unobtrusively hidden by the material of the reins and form part of the visual continuity of the reins. The cable carrying signals to the transceiver can be also embedded into the reins thus improving the visual continuity and helping prevent the possibility of trapping in the bridlework when in use. To assist with the embedding of the rein tension sensor unit it ca be provided with mechanical attachments one at each of opposite ends that are provided with suitable holes for fastening directly and permanently to the material of the reins such as nylon webbing or other material so that there is no need for clips or bolts on the rein tension sensor unit to which the reins attach. These features make the rein tension sensor unit more readily usable on a regular basis and less likely to be objectionable to judges of the governing bodies of the equestrian sports.

## Detailed description of preferred embodiments

In the following the invention will be explained, by way of preferred embodiments, in more detail with reference to the drawings, wherein

Fig. 1    shows a horse's head with part of the apparatus of the invention;

Fig. 2    is an exploded plan view of part of the apparatus of Fig.1;

Fig. 3    is a plan view of a rein tension sensor unit of the apparatus of Fig.1

Fig. 4    shows a schematic diagram of part of the apparatus;

Fig. 5    shows a graph indicating a power spectral density PSD$(f)$ versus frequency $f$ and further parameters of the method of the invention;

Fig. 6    shows an enlarged section of the graph of Fig. 6;

Fig. 7    depicts a graphical representation of a look-up table where a Figure of Merit $FoM$ can be assigned to an average force value $\overline{F}_R$ measured by the apparatus of the invention.

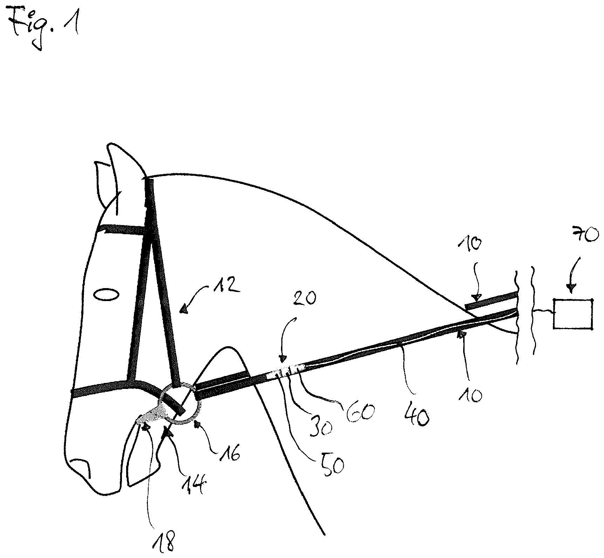

Fig. 1 shows a horse's head and a bridle 12 mounted on the horse's head. The bridle 12 holds a bit 14 which has two side rings 16 and a mouthpiece 18. A first side ring 16 is positioned on the left side of the horse's head and a second side ring 16 is positioned on the right side of the horse's head as seen by a rider positioned on the horse. The mouthpiece 18 is inserted into the horse's mouth and extends between the two side rings 16. Further, reins 10 are connected to each of the side rings 16. Thus, one of the reins 10 is mounted to the first side ring 16 and the other one of the reins 10 is mounted to the second side ring 16. The rider holds the reins 10 for giving commands to the horse. When pulling the reins 10 a force is transmitted via the reins 10 to the side ring and subsequently to the mouthpiece 18 of the bit 14.

To measure the force in the reins each rein 10 comprises a rein tension measuring device 20 as a part of the apparatus of the invention. The rein tension measuring device 20 comprises a rein tension sensor unit 30 which can also be designated as force transducer or strain gauge.

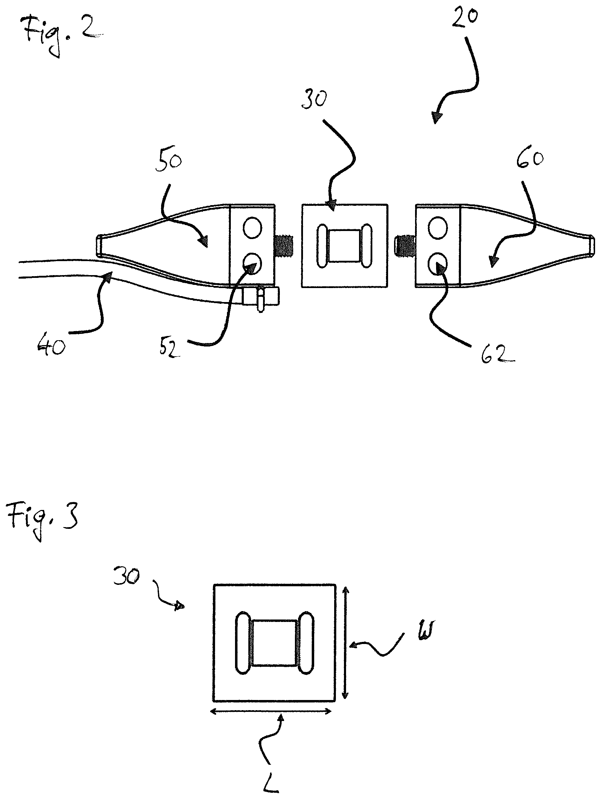

Fig. 2 depicts an exploded plan view of the rein tension measuring device 20 showing the rein tension sensor unit 30 and attachment units 50, 60 with holes 52, 62 for fastening to the reins 10 by a suitable fastener. The rein tension sensor unit or force transducer 30 is placed between the attachment units 50, 60. Further, an electric cable 40 is provided for transmitting signals from the rein tension sensor unit 30 to a transmitter unit 70.

The rein tension measuring device 20 is inserted into each of two reins 10 one on each side of the animal so that a time-series signal can be measured from each which is proportional to the time-series rein tension on each side of the animal. The rein tension sensor units 30 are hidden from view by being fully encased in the construction material of the reins 10. Preferably, also the attachment units 50, 60 are embedded and encased in the material of the reins 10.

Referring to Fig. 3 the rein tension sensor unit 30 is preferably smaller than 50 mm in length L and width W and less than 15 mm in depth. In a further preferred embodiment the rein tension sensor unit 30 is preferably smaller than 20 mm in length W and width W and less than 10 mm in depth. In another preferred embodiment the rein tension sensor unit 30 is 19 mm in length W, 16 mm in width W and 6 mm in depth.

Preferably, each end of the rein tension sensor unit 30 is provided with suitable mechanical attachment units 50, 60 with holes 52, 62 so that it can be attached to the construction material of the reins 10 using a suitable fastener. A preferred fastener is a rivet fastener but alternatives are possible.

Also preferred the electrical cable 40 is hidden from view by being fully encased in the construction materials of the reins.

It is known that force transducers can be mounted to the reins but with the disadvantage of visual discontinuity. The transducers of the prior art attach to the reins or bridlework using clips or hooks and are designed for temporary use. These items make regular use of the transducers difficult and further distract from the desirable visual continuity of traditional reins and bridlework. A further disadvantage is that the electrical cables which are necessary to carry signals from the force transducer to the transmitter are also visible and obtrusive and may become trapped in the bridlework or by the rider's hands during riding.

The rein tension sensor unit 30 and electric cable 40 is preferably of a small form factor and consequently of light weight so that it can be unobtrusively hidden by the material of the reins 10 and form part of the visual continuity of the reins. The cable carrying signals to the transceiver is also embedded into the reins thus improving the visual continuity and helping prevent the possibility of trapping in the bridlework when in use. To assist with the embedding of the rein tension sensor unit 30 it is provided with mechanical attachments one at each of opposite ends that are provided with suitable holes for fastening directly and permanently to the material of the reins such as nylon webbing or other material so that there is no need for clips or bolts on the rein tension sensor unit 30 to which the reins 10 must attach. These features make the rein tension sensor unit 30 more readily usable on a regular basis and less likely to be objectionable to judges of the governing bodies of the equestrian sports.

The rein tension sensor unit 30 on each side of the animal send the measured rein tension data via the transmitter unit 70 to a receiving unit 80. Subsequently, the received data can be processed using a processing unit 90. Preferably, each pair of signals is sampled within a particular span of time and is recorded and stored for post-processing. The members of the pair of signals can be labelled Left and Right for example. Each data set comprises a time-ordered list of Left and Right signals whose magnitude represents the force acting in the reins at particular intervals of time. Each list is called a time-series.

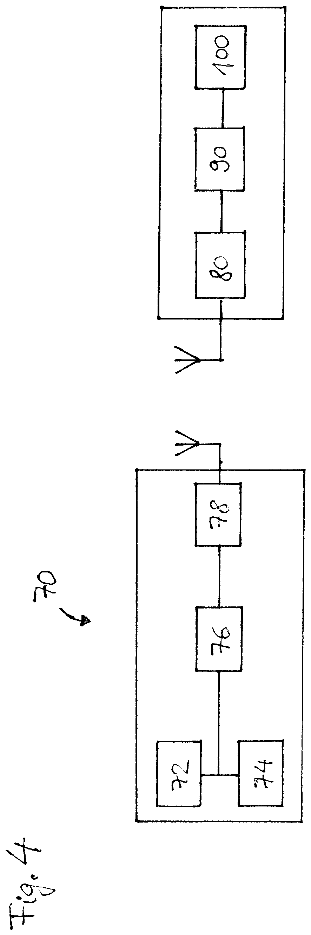

The transmitter unit 70 can comprise at least one data collecting unit, an amplifier, an A/D converter and/or a wireless transmitter. As shown schematically in Fig. 4, the transmitter unit 70 comprises a first data collecting unit 72 for collecting the rein tension data of the first (or left-side) rein tension unit 30 and second data collecting unit 74 for collecting the rein tension data of the first (or left-side) rein tension unit 30. Further, a data converting unit 76 is provided comprising an amplifier and an A/D converter for converting the data into suitable signals. Further, the transmitter unit 70 comprises a wireless transmitter 78 for wireless transmitting the signals to the receiving unit 80 for further processing by the processing unit 90. Finally, the processed data can be notified to a display unit 100 for informing a user about the quality of characteristics of the motion of the horse-rider-dyad.

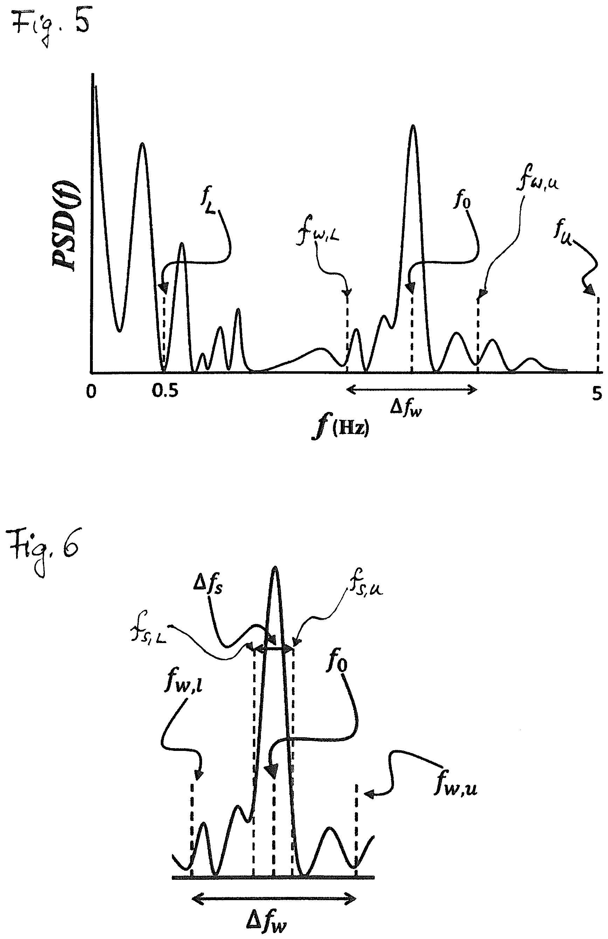

Based on Fig. 5 and 6, in the following a preferred embodiment of the method of the invention for evaluating the quality of characteristics of motion of an animal-human-dyad between an animal and a human using a rein 10 for riding the animal is described. In a first step rein tension data indicating the tension in the rein 10 is obtained. Preferably, the rein tension in the rein 10 is measured by at least one strain gauge inserted into the at least one rein 10. In a second step the rein tension data is processed into a frequency spectrum $f$ (Hz). In a third step a prominent frequency component $f_0$ in the frequency spectrum is identified. In a fourth step the frequency spectrum is analysed by integrating the power spectral density PSD($f$) of the frequency spectrum over a frequency range $\Delta f_s$ which contains the prominent frequency component $f_0$. Finally, the quality of characteristics of motion is evaluated by comparing the integral of the power spectral density PSD($f$) value determined over the range $\Delta f_s$ with a reference value found by integrating over a frequency range $\Delta f_w$.

Preferably, the frequency spectrum is analysed only within an overall frequency range limited by a first frequency threshold $f_L$ and a second frequency threshold $f_U$. For example, the first frequency threshold $f_L$ is about 0.5 Hz and the second frequency threshold $f_U$ is about 5.0 Hz. Preferably, the frequency spectrum is analysed within a frequency sub-range $\Delta f_S$ spanning the prominent frequency component. By analysing the frequency spectrum over the frequency range $\Delta f_W$ and the frequency sub-range $\Delta f_S$ preferably a signal-to-noise ratio (s/n) is determined. Preferably, the reference value is the prominent frequency component $f_0$ and/or the reference value is a value that correlates to the stride frequency $f_C$ or $f_T$ or gait frequency of the animal or the second harmonic of that frequency.

In the following a preferred embodiment of the method of the invention is described. The preferred embodiment of the method includes the determination of the frequency-dependent power spectral density $PSD(f)$ of each of the Left and Right time-series rein tension data and the identification of the prominent frequency component $f_0$ of the power spectral density $PSD(f)$ within a (restricted) frequency range $\Delta f_W$ that contains the stride frequency or multiple thereof for a particular mode of motion of the animal, for example $\Delta f_C$ or $\Delta f_T$, that itself lies within a larger restricted frequency range having upper and lower frequency limits $f_U$ and $f_L$ respectively.

A suitable small range of frequencies, $\Delta f_s$ spanning the centre frequency is then defined and the integral of PSD($f$), $I_s$ between the upper $f_{s,u}$ and lower $f_{s,l}$ limits of this range is computed. This integral can be represented as:

$$I_s = \int_{f_{s,l}}^{f_{s,u}} PSD(f).df \tag{1}$$

Included in the method is the computation of the integral of PSD($f$) over a wider range of frequencies, $\Delta f_w$ spanning $f_0$ with upper and lower frequency limits $f_{w,u}$ and $f_{w,l}$, respectively. This integral can be represented as

$$I_w = \int_{f_{w,l}}^{f_{w,u}} PSD(f).df \tag{2}$$

The final step in the method is to calculate the ratio, $R$ of these two integrals as

$$R = \frac{I_s}{I_w} \tag{3}$$

The lower limit of $R$ depends on the spectral distribution of noise in the calculated range between frequency limits $f_{w,u}$ and $f_{w,l}$.

In the specific example where there can be considered to be so-called white noise $R$ becomes a number lying between $\Delta f_s / \Delta f_w$, being the lowest value, and 1.

Fig. 5 shows the power spectral density *PSD(f)* versus frequency $f$ and parameters of the method of the invention, wherein the parameters include the prominent frequency $f_0$, the larger restricted frequency range upper and lower limits $f_U$ and $f_L$ and the wider range of frequencies $\Delta f_w$ over which the computation of the integral of the *PSD(f)* is carried out.

Fig. 6 shows further the parameters of the method of the invention $f_{w,u}$, $f_{w,l}$, $f_{s,l}$, $f_{su}$, $\Delta f_s$

Preferably $\Delta f_w$ is around 1.4 Hz and $\Delta f_s$ is around 0.6 Hz but other values are possible which will be apparent to those skilled in the art.

The values of $f_{w,w}$, $f_{w,l}$, $f_{s,l}$, $f_{su}$, are chosen according to the mode of motion of the animal.

Amongst the various modes of motion of 4 legged animals are the modes walk, trot and canter.

For walk, preferably $f_{w,u}$ and $f_{w,l}$ are around 4.6 and 2.6 Hz, respectively although other values are possible.

For trot, preferably $f_{w,u}$ and $f_{w,l}$ are around 4.4 and 2.4 Hz, respectively although other values are possible.

For canter, preferably $f_{w,u}$ and $f_{w,l}$ are around 2.2 and 1.2 Hz, respectively although other values are possible.

Other modes of motion are possible and values of $f_{w,u}$ and $f_{w,l}$ can then be chosen appropriately by those skilled in the art.

The ratio $R$ represents a definition of the signal-to-noise ratio $\frac{s}{n}$ of the *PSD* of a general time-series signal and that a value $R = 1$ indicates that there is no noise within the measured range of frequencies. Conversely where $R = \Delta f_s / \Delta f_w$ all is white noise within the measured range of frequencies and the signal if present becomes part of that noise.

A measure of quality of the characteristics of motion of the horse/rider dyad is the qualitative sensation in the rider of an irregularity in the otherwise ideal rhythmical tension in the reins. When judging the equestrian sports by observation of the motion of the horse/rider dyad the observed quality of the characteristics of motion of the horse/rider dyad is measured as a perceived loss of ideal regularity of rhythm in the motion.

Using the method of the invention it can then be made objective that a perceived loss of regularity in the rhythm of the motion of an animal/human dyad or the sensation by the human of irregularity in the rhythmical tension in the reins can be quantified as a value in the s/n.

Regarding the ranges of frequencies, the choice of limits $f_U$ and $f_L$ are preferably 5 Hz and 0.5 Hz ($f_5$ and $f_{0.5}$).

It is advantageous to the application of the method of the invention to ignore contributions to the PSD that lie below 0.5 Hz as these contributions may be regarded as arising from quasi-random events such as instantaneous corrective impulses applied from the rider through the reins to the horse that are usually acceptable to judges of the governing bodies of the equestrian sports. Similarly, it is advantageous to the application of the method of the invention to ignore contributions to the PSD that lie above 5 Hz as these are due to random noise that is unconnected with the measurement of the quality of motion of the dyad as will now be explained.

The frequency range $\Delta f_W$ must be chosen suitably so that the stride frequency or multiple thereof of the animal lies within them. The stride frequency of the animal depends on its physiology and mode of motion.

For example one mode of motion of a four legged animal is called canter and in the horse a typical frequency at canter $f_C$ lies within a range $1.2Hz \le f_C \le 2.2Hz$ making $\Delta f_C$, = $1Hz$.

A further example of a mode of motion of a four legged animal is called trot and in the horse a typical frequency at trot $f_T$ also lies within a range $1.2Hz \le f_T \le 2.2Hz$ making $\Delta f_T$ = $1Hz$.

Further advantageous in the application of the method is to choose a range $\Delta f_{2,T}$ spanning the doubled frequency of the typical stride frequency of trot which is called the second harmonic frequency $f_{2,T}$ so that $2.4Hz \le f_{2,T} \le 4.4Hz$ making $\Delta f_{2,T} = 2Hz$

It is further advantageous to choose a range $\Delta f_{2,W}$ spanning the doubled frequency of the typical stride frequency of walk which is called the second harmonic frequency $f_{2,W}$ so that $2,6Hz \le f_{2,W} \le 4.6Hz$ making $\Delta f_{2,W} = 2Hz$

Preferably, the method can be used to compute the PSD ratio in trot at the stride frequency $R_T$ and also at the second harmonic frequency $R_{2,T}$.

By further application of the method particularly when the mode of motion of the horse is trot it is possible to

$$P = {R_{2,T}}/{R_T}$$

compute the ratio $P$ of the values $R_T$ and $R_{2,T}$ so that which lies between values of zero and infinity.

When $P$ is high there is indicated an unevenness in the full stride at trot of the horse which is a condition called laterality.

When the horse has no such condition such a high ratio of $R_T$ to $R_{2,T}$ could also indicate a rhythmical unleven application by the rider of forces to the reins. This is also called laterality. When there is no such laterality present the ratio will be zero.

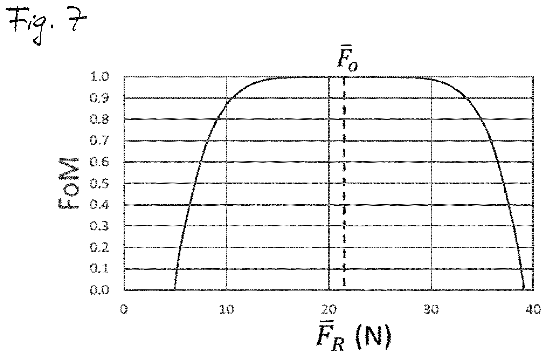

A further method of the invention, referring to Fig. 7, includes the computation of the average force in the reins $\overline{F}_R$ for each of the Left and Right time-series data and comparison of those values with a look-up table defining requirements for success in the equestrian sports. Requirements for success might be stipulated by the governing bodies that define and organise the equestrian sports and might include requirements that the average force in the reins must lie within an acceptable range of values spanning an ideal central value $\overline{F}_o$.

By way of example one such look-up table could comprise the definition of a figure of merit FoM whose maximum value is an optimum average tension in the reins $\overline{F}_o$ which is kept substantially constant within a range of higher or lower average rein tensions to either side of $\overline{F}_o$.

Further higher or lower to either side of the optimum average rein tension the FoM attenuates progressively to zero.

Preferably, a look-up table is provided giving a figure of merit *FoM* being a value lying between 0 and 1 versus the independent variable $\overline{F}_R$ (see Figure 7).

Fig. 7 depicts a graphical representation of a look-up table of the invention where a Figure of Merit *FoM* can be assigned to an average force value $\overline{F}_R$ measured by the apparatus of the invention. The defined optimum average force in the reins $\overline{F}_o$ indicated in the figure is 22 N and the range in the independent variable to either side where the *FoM* is substantially optimised lies between around 15 N and 30 N.

One example of an algorithm for providing a look-up table is given as

$$FoM_{L,R}(\phi) = 1 - \left[ P\cos^{-2}(\phi)\{\sin^3(\phi)\}^2 exp[-C(\cos^{-1}(\phi))] \right] \tag{4}$$

wherein $FoM_{L,R}(\phi)$ is the figure of merit of the parameter $\phi$ for comparison with either the Left or Right average rein tension and $P$ and $C$ are constants. The parameter $\phi$ gives a suitable scale for the independent variable and is given as

$$\phi = \frac{F_R - F_o}{W} \tag{5}$$

wherein $W$ is a suitably chosen variable that governs the overall range or width of applicability of the independent variable.

By way of example the particular form of the *FoM* depicted in Fig 7 where the force units are given in Newtons has $\overline{F}_o$ = 22 , $P$ = 26.5, $C$ = 0.8 and $W$ = 25

The $\overline{F}oM$ just described may be multiplied with $R$ to give a better overall objective indication of the quality of the characteristics of motion of the horse/rider dyad.

A further method of the invention is the computation of the ratio of the average rein tension between the left and right time series average rein tensions and a method to provide a figure of merit that is a maximum when that ratio is 1. This further figure of merit can be further multiplied with $R$ and *FoM* to give a more refined measure of the quality of the characteristics of motion of the horse/rider dyad.

**List of Reference Signs**

| | |
|---|---|
| 10 | rein |
| 12 | bridle |
| 14 | bit |
| 16 | side ring |
| 18 | mouthpiece |

| | |
|---|---|
| 20 | rein tension measuring device |
| 30 | Rein tension sensor unit |
| 40 | electric cable |
| 50 | attachment unit |
| 52 | attachment hole |
| 60 | attachment unit |
| 62 | attachment hole |

| | |
|---|---|
| 70 | transmitter unit |
| 72 | first data collecting unit |
| 74 | second data collecting unit |
| 76 | data converting unit |
| 78 | wireless transmitter |

| | |
|---|---|
| 80 | receiving unit |
| 90 | processing unit |
| 100 | notification unit (display unit) |

| | |
|---|---|
| L | length |
| W | width |

**Claims**

1. Method for evaluating the quality of characteristics of motion of an animal-human-dyad comprising an animal and a human and using at least one rein (10) for riding the animal, wherein the quality of characteristics of motion is the loss of regularity of rhythm in the motion of the dyad, the method comprising the following step:

   obtaining rein tension data indicating the tension in the rein (10) by means of a rein tension measuring device (20);
   **characterized by** the following steps executed by a processing unit (90):

   processing the rein tension data into a frequency spectrum;
   identifying a prominent frequency component in the frequency spectrum;
   analysing the frequency spectrum by determining a power spectral density of the frequency spectrum over a frequency range which contains the prominent frequency component; and
   evaluating the loss of regularity of rhythm in the motion by comparing the determined power spectral density with a reference value.

2.  Method according to claim 1, **characterized in that** the frequency spectrum is analysed within a frequency sub-range spanning the prominent frequency component.

3.  Method according to any of claims 1 or 2, **characterized in that** the frequency spectrum is analysed only within an overall frequency range limited by a first frequency threshold and a second frequency threshold.

4.  Method according to any of the preceding claims, **characterized in that** the frequency range and/or a frequency sub-range is analysed to determine a signal-to-noise ratio (s/n).

5.  Method according to claim 4, **characterized in that** the signal-to-noise ratio of at least the prominent frequency component is determined.

6.  Method according to any of claims 4 or 5, **characterized in that** the signal-to-noise ratio of two or more prominent frequency components are determined and that a ratio of their values is determined.

7.  Method according to any of the preceding claims, **characterized in that** the reference value is the prominent frequency component and/or the reference value is a value that correlates to the stride frequency or gait frequency of the animal or the second harmonic of the stride frequency or gait frequency of the animal.

8.  Method according to any of the preceding claims, **characterized in that** obtaining rein tension data indicating the tension in the rein (10) comprises measuring the rein tension by at least one strain gauge inserted into the rein (10).

9.  Method according to any of the preceding claims, **characterized by** determining an average of the rein tension using the rein tension data and comparing with a reference value or reference table of rein tension values.

10. Apparatus for evaluating the quality of characteristics of motion of an animal-human-dyad comprising an animal and a human and using at least one rein (10) for riding the animal, wherein the quality of characteristics of motion is the loss of regularity of rhythm in the motion of the dyad, the apparatus comprising:

    at least one rein tension measuring device (20) configured to obtain rein tension data;
    a transmitter unit (70) configured to transmit the rein tension data;
    a receiving unit (80) configured to receive the data;
    a processing unit (90) configured to process the rein tension data into a frequency spectrum, to identify a prominent frequency component in the frequency spectrum, to analyse the frequency spectrum by determining the power spectral density over a frequency range which contains the prominent frequency, and to evaluate the loss of regularity of rhythm in the motion by comparing the determined power spectral density with a reference value, and
    a notification unit (100) for notifying about the loss of regularity of rhythm in the motion based on the processed data.

11. Apparatus according to claim 10, **characterized in that** the processing unit (90) is configured to process the data according to any of the claims 2 to 9.

12. Apparatus according to any of claims 10 or 11, **characterized in that** the rein tension measuring device (20) is embedded in the rein (10).

13. Apparatus according to any of claims 10 to 12, **characterized in that** an electric cable (40) for transmitting signals from the rein tension measuring device (20) to the transmitting unit is embedded in the rein (10).

**Patentansprüche**

1.  Verfahren zum Auswerten der Qualität der Bewegungscharakteristik einer Tier-Mensch-Dyade umfassend ein Tier und ein Mensch und mindestens einen Zügel (10) verwendend, um das Tier zu reiten, wobei die Qualität der Bewegungscharakteristik der Verlust der Regelmäßigkeit des Rhythmus der Bewegung der Dyade ist, wobei das Verfahren die folgenden Schritte aufweist:
    Erhalten der Zügelzugkraftdaten, welche die Zugkraft in dem Zügel (10) mittels einer Zügelzugkraftmessvorrichtung (20) angibt;

**gekennzeichnet durch** die folgenden Schritte, die durch eine Verarbeitungseinheit (90) ausgeführt werden:

Verarbeiten der Zügelzugkraftdaten in ein Frequenzspektrum;
Identifizieren einer auffälligen Frequenzkomponente in dem Frequenzspektrum;
Analysieren des Frequenzspektrums durch Ermitteln einer spektralen Leistungsdichte des Frequenzspektrums über einen Frequenzbereich, der die auffällige Frequenzkomponente umfasst; und
Auswerten des Verlusts der Regelmäßigkeit des Rhythmus der Bewegung, indem die ermittelte spektrale Leistungsdichte mit einem Referenzwert verglichen wird.

2. Verfahren nach Anspruch 1, **dadurch gekennzeichnet, dass** das Frequenzspektrum in einem Teilfrequenzbereich analysiert wird, der die auffällige Frequenzkomponente umfasst.

3. Verfahren nach Anspruch 1 oder 2, **dadurch gekennzeichnet, dass** das Frequenzspektrum nur innerhalb eines Gesamtfrequenzbereichs analysiert wird, welcher von einem ersten Frequenzgrenzwert und einen zweiten Frequenzgrenzwert limitiert wird.

4. Verfahren nach einem der vorhergehenden Ansprüche, **dadurch gekennzeichnet, dass** der Frequenzbereich und/ oder ein Teilfrequenzbereich zur Bestimmung eines Signal-Rausch-Verhältnisses (s/n) analysiert wird.

5. Verfahren nach Anspruch 4, **dadurch gekennzeichnet, dass** das Signal-Rausch-Verhältnis (s/n) von mindestens der auffälligen Frequenzkomponente bestimmt wird.

6. Verfahren nach Anspruch 4 oder 5, **dadurch gekennzeichnet, dass** das Signal-Rausch-Verhältnis (s/n) von zwei oder mehr auffälligen Frequenzkomponenten bestimmt wird und dass ein Verhältnis von diesen Werten bestimmt wird.

7. Verfahren nach einem der vorhergehenden Ansprüche, **dadurch gekennzeichnet, dass** der Referenzwert die auffällige Frequenzkomponente und/oder der Referenzwert ein Wert ist, welcher mit der Schrittfrequenz oder Gangfrequenz des Tieres oder der zweiten Oberschwingung der Schrittfrequenz oder Gangfrequenz des Tieres korreliert.

8. Verfahren nach einem der vorhergehenden Ansprüche, **dadurch gekennzeichnet, dass** das Erhalten der Zügelzugkraftdaten, welche die Zugkraft in dem Zügel (10) angibt, ein Messen der Zügelkraft mittels mindestens eines Dehnungsmessers, der in dem Zügel (10) eingefügt ist, umfasst.

9. Verfahren nach einem der vorhergehenden Ansprüche, **gekennzeichnet durch** Ermitteln eines Durchschnitts der Zügelzugkraft, indem die Zügelzugkraftdaten verwendet werden und mit einem Referenzwert oder einer Referenztabelle von Zügelzugkraftwerten verglichen wird.

10. Vorrichtung zum Auswerten der Qualität der Bewegungscharakteristik einer Tier-Mensch-Dyade umfassend ein Tier und ein Mensch und mindestens einen Zügel (10) verwendend, um das Tier zu reiten, wobei die Qualität der Bewegungscharakteristik der Verlust der Regelmäßigkeit des Rhythmus der Bewegung der Dyade ist, wobei die Vorrichtung aufweist:

mindestens eine Zügelzugkraftmessvorrichtung (20), die ausgebildet ist Zügelzugkraftdaten zu erfassen;
eine Transmittereinheit (70), die ausgebildet ist, die Zügelzugkraftdaten zu übertragen;
eine Empfangseinheit (80), die ausgebildet ist, die Daten zu empfangen;
eine Verarbeitungseinheit (90), die ausgebildet ist, die Zügelzugkraftdaten in ein Frequenzspektrum zu verarbeiten, um eine auffällige Frequenzkomponente in dem Frequenzspektrum zu identifizieren, um das Frequenzspektrum durch die Ermittlung der spektralen Leistungsdichte über einen Frequenzbereich, welcher die auffällige Frequenz beinhaltet, zu analysieren, und um den Verlust der Regelmäßigkeit des Rhythmus der Bewegung durch Vergleichen der ermittelten spektralen Leistungsdichte mit einem Referenzwert auszuwerten, und
eine Benachrichtigungseinheit (100) zum Benachrichtigen über den Verlust der Regelmäßigkeit des Rhythmus der Bewegung basierend auf den verarbeiteten Daten.

11. Vorrichtung nach Anspruch 10, **dadurch gekennzeichnet, dass** die Verarbeitungseinheit (90) ausgebildet ist, Daten nach einem der Ansprüche 2 bis 9 zu verarbeiten.

12. Vorrichtung nach Anspruch 10 oder 11, **dadurch gekennzeichnet, dass** die Zügelzugkraftmessvorrichtung (20) in dem Zügel (10) eingebettet ist.

**13.** Vorrichtung nach einem Ansprüche 10 bis 12, **dadurch gekennzeichnet, dass** ein elektrisches Kabel (40) zur Übertragung von Signalen von der Zügelzugkraftmessvorrichtung (20) zu der Transmittereinheit in dem Zügel (10) eingebettet ist.

## Revendications

**1.** Procédé d'évaluation de la qualité de caractéristiques de mouvement d'une dyade animal-humain comprenant un animal et un humain et utilisant au moins une rêne (10) pour monter l'animal, dans lequel la qualité de caractéristiques de mouvement est la perte de régularité de rythme dans le mouvement de la dyade, le procédé comprenant l'étape suivante :

obtenir de données de tension de rêne indiquant la tension dans le rêne (10) au moyen d'un dispositif de mesure de tension de rêne (20) ;
**caractérisé par** les étapes suivantes exécutées par une unité de traitement (90) ;
traiter les données de tension de rêne en un spectre de fréquences ;
identifier une composante de fréquence prépondérante dans le spectre de fréquences ;
analyser le spectre de fréquences en déterminant une densité spectrale de puissance du spectre de fréquences sur une gamme de fréquences qui contient la composante de fréquence prépondérante ; et
évaluer la perte de régularité de rythme dans le mouvement par comparaison de la densité spectrale de puissance déterminée avec une valeur de référence.

**2.** Procédé selon la revendication 1, **caractérisé en ce que** le spectre de fréquence est analysé dans une sous-gamme de fréquences couvrant la composante de fréquence prépondérante.

**3.** Procédé selon l'une quelconque des revendications 1 ou 2, **caractérisé en ce que** le spectre de fréquences n'est analysé que dans une gamme de fréquences globale limitée par un premier seuil de fréquence et un second seuil de fréquence.

**4.** Procédé selon l'une quelconque des revendications précédentes, **caractérisé en ce que** la gamme de fréquences et/ou une sous-gamme de fréquences est analysée pour déterminer un rapport signal/bruit (s/n).

**5.** Procédé selon la revendication 4, **caractérisé en ce que** le rapport signal/bruit d'au moins la composante de fréquence prépondérante est déterminé.

**6.** Procédé selon l'une quelconque des revendications 4 ou 5, **caractérisé en ce que** le rapport signal/bruit de deux ou plusieurs composantes de fréquence prépondérante est déterminé et **en ce qu'**un rapport de leurs valeurs est déterminé.

**7.** Procédé selon l'une quelconque des revendications précédentes, **caractérisé en ce que** la valeur de référence est la composante de fréquence prépondérante et/ou la valeur de référence est une valeur qui est en corrélation avec la fréquence de foulée ou à la fréquence de marche de l'animal ou à la seconde harmonique de la fréquence de foulée ou de la fréquence de marche de l'animal.

**8.** Procédé selon l'une quelconque des revendications précédentes, **caractérisé en ce que** l'obtention de données de tension de rêne indiquant la tension dans le rêne (10) comprend la mesure de la tension du rêne par au moins une jauge de contrainte insérée dans le rêne (10).

**9.** Procédé selon l'une quelconque des revendications précédentes, **caractérisé par** la détermination d'une moyenne de la tension du rêne en utilisant les données de tension de rêne et en la comparant avec une valeur de référence ou un tableau de référence des valeurs de tension de rêne.

**10.** Appareil d'évaluation de la qualité des caractéristiques de mouvement d'une dyade animal-humain comprenant un animal et un humain et utilisant au moins une rêne (10) pour monter l'animal, dans lequel la qualité de caractéristiques de mouvement est la perte de régularité de rythme dans le mouvement de la dyade, l'appareil comprenant :

au moins un dispositif de mesure de tension de rêne (20) configuré pour obtenir des données de tension de rêne ;

une unité de transmission (70) configurée pour transmettre les données de tension de rêne ;

une unité de réception (80) configurée pour recevoir les données ;

une unité de traitement (90) configurée pour traiter les données de tension de rêne dans un spectre de fréquences, pour identifier une composante de fréquence prépondérante dans le spectre de fréquences, pour analyser le spectre de fréquences en déterminant la densité spectrale de puissance sur une gamme de fréquences qui contient la fréquence prépondérante, et pour évaluer la perte de régularité de rythme dans le mouvement en comparant la densité spectrale de puissance déterminée avec une valeur de référence, et

une unité de notification (100) pour notifier la perte de régularité de rythme dans le mouvement, sur la base des données traitées.

11. Appareil selon la revendication 10, **caractérisé en ce que** l'unité de traitement (90) est configurée pour traiter les données selon l'une quelconque des revendications 2 à 9.

12. Dispositif selon l'une quelconque des revendications 10 ou 11, **caractérisé en ce que** le dispositif de mesure de tension de rêne (20) est intégré dans le rêne (10).

13. Dispositif selon l'une quelconque des revendications 10 à 12, **caractérisé en ce qu'**un câble électrique (40) pour transmettre des signaux du dispositif de mesure de tension de rêne (20) à l'unité de transmission est intégré dans le rêne (10).

Fig. 1

Fig. 2

Fig. 3

Fig. 4

Fig. 5

Fig. 6

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5369601 A
- NZ 540119 A

- US 20150106052 A1